# EUROPEAN PATENT SPECIFICATION

(11) **EP 4 472 429 B1**
(45) Date of publication and mention of the grant of the patent: **26.11.2025**
(21) Application number: 23706694.9
(22) Date of filing: 26.01.2023
(51) Int. Cl.: A23D 9/02, C11B 1/04, C11B 1/10

(54) **SUNFLOWER OLEOSOME CONCENTRATE AND PROCESS FOR PREPARING IT**
SONNENBLUMEN-OLEOSOMKONZENTRAT UND VERFAHREN ZU SEINER HERSTELLUNG
CONCENTRÉ D'OLÉOSOMES DE TOURNESOL ET SON PROCÉDÉ DE PRÉPARATION

(30) Priority: 03.02.2022 EP 22155054
(43) Date of publication of application: 11.12.2024
(73) Proprietor: Cargill, Incorporated, Wayzata, Minnesota 55391 (US)
(72) Inventor: CABAS-RODRIGUEZ, Lucía, 2060 Antwerpen (BE); GOOSSENS, Eliane Yvonne, 2845 Niel (BE); WASCHATKO, Gustav Maximilian, 1040 Etterbeek (BE)
(74) Representative: Elseviers, Myriam
(86) International application number: PCT/US2023/061357
(87) International publication number: WO 2023/150462

(56) References cited:
- WO-A1-2021/126407
- WO-A1-2021/126408
- WO-A1-2021/126409
- WO-A1-2021/145941

## Description

### CROSS-REFERENCE TO RELATED APPLICATION

### FIELD OF THE INVENTION

The invention relates to a process for isolating a sunflower oleosome concentrate from sunflower seeds. The invention further relates to a sunflower oleosome concentrate, and a process for preparing food and feed products, pharmaceutical products, personal care products, nutritional compositions and industrial products comprising said sunflower oleosome concentrate.

### BACKGROUND OF THE INVENTION

Oleosomes, also known as "oil bodies", "lipid bodies", "lipid droplets" or "spherosomes", are pre-emulsified droplets or vesicles of oil stored in plant seeds and used as energy source for plant growth and metabolism.

Oleosomes are typically extracted from cells by a process of grinding the seeds, followed by solid-liquid separation to obtain an aqueous suspension of oleosomes. Said suspension is centrifuged to separate the oleosomes from the remaining seed content and obtain an oleosome concentrate.

It is important that the process conditions for isolating oleosome concentrates are such that the yield of oleosome concentrates obtained from the oil seeds is maximised. Unfortunately, while maximizing yield, the oleosome concentrates obtained according to existing processes are less robust, have a less interesting nutritional profile and a less creamy sensorial effect. This is especially the case when isolating oleosome concentrates from sunflower seeds.

Therefore, there is a need in industry to identify an efficient and effective method for obtaining sunflower oleosome concentrates with improved qualities in terms of robustness, nutritional profile and sensorial characteristics. WO 2021/145941 A1 describes a process for increasing the yield of an isolated oleosome composition and the process is comprising blending of a processed fiber fraction of an oleosome source with at least one other fraction of an oleosome source.

### SUMMARY OF THE INVENTION

The current invention relates to a process for preparing a sunflower oleosome concentrate and the process is comprising the steps, in order, of:
a) Grinding sunflower seeds in the presence of an aqueous solution and obtaining a sunflower seed slurry having a pH of from 7.0 to 7.2, and
b) Isolating from the sunflower seed slurry a sunflower oleosome fraction, and
c) Washing the sunflower oleosome fraction at a pH in a range of from 6.6 to 7.0 and isolating the sunflower oleosome concentrate.

The invention also relates to a sunflower oleosome concentrate that is characterized in that it has based on total dry matter:
- a protein content of from 8.0 to 17.0 wt.%: and
- a lipid content of from 83 to 92 wt.%, of which at least 70 wt.% is present as oleosomes, wherein the sunflower oleosome concentrate has a color, expressed in color values according to CIELAB of: °h in a range of from 85 to 95, from 86 to 94, or from 87 to 93 and C* of from 8.0 to 14.0, from 8.2 to 13.8, or from 8.4 to 13.6; or °h above 95, above 96, or above 97, and C* below 10.0, below 9.8, or below 9.6; measured on sunflower oleosome concentrate standardized to a dry substance in a range of 30 to 35 % and a pH in a range of 6.9 to 7.1, using an illuminant D65, an observation angle of 10° and an aperture size of 30mm.

Furthermore, the invention relates to a process for preparing food and feed products, pharmaceutical products, personal care products, nutritional compositions and industrial products, and the process is comprising the step of blending the sunflower oleosome concentrate according to the invention with at least one ingredient other than oleosomes.

### DETAILED DESCRIPTION

The current invention relates to a process for preparing an isolated sunflower concentrate and the process is comprising the steps, in order, of:
a) Grinding sunflower seeds in the presence of an aqueous solution and obtaining a sunflower seed slurry having a pH of from 7.0 to 7.2, and
b) Isolating from the sunflower seed slurry a sunflower oleosome fraction, and
c) Washing the sunflower oleosome fraction at a pH in a range of from 6.6 to 7.0 and isolating the sunflower oleosome concentrate.

For the process of the current invention any type of sunflower seeds, belonging to the species *Helianthus annuus,* may be used.

Several types of sunflower seeds exist, each characterized by the fatty acid profile of the oil present in these seeds. Sunflower seeds contain sunflower oil that is characterized by a typical profile of 66 to 72% linoleic acid, 9 to 13% saturated fatty acids (palmitic and stearic), 17 to 23% oleic acid, and less than 1% alpha-linolenic acid.

Other well-known varieties of sunflower seeds are mid- and high oleic sunflower seeds. Typically, a high-oleic sunflower oil is characterized by a content of 7 to 11% linoleic acid, 7 to 11% saturated fatty acids (palmitic and stearic), 80 to 86% oleic acid, and less than 1% alpha-linolenic acid. Typically, a mid-oleic sunflower oil is characterized by a content of 24 to 28% linoleic acid, 7 to 11% saturated fatty acids (palmitic and stearic), 62 to 68% oleic acid, and less than 1% alpha-linolenic acid.

Preferably the sunflower seeds used in the process according to the present invention are mid- or high-oleic sunflower seeds; more preferably high-oleic sunflower seeds.

The process steps a) to c) of the process according to the invention, and optionally process steps of soaking and washing, are performed at a temperature that does not exceed 60°C, preferably 55°C, more preferably 50°C

Furthermore, the process steps a) to c) of the process according to the invention, and optionally process steps of soaking and washing, are performed in absence of an organic solvent. The organic solvent may be acetone, hexane, ethanol and the like.

### Step a) Obtaining a sunflower seed slurry having pH of from 7.0 to 7.2

A sunflower seed slurry is obtained in step a) of the process according to the invention.

The sunflower seed slurry is a slurry comprising oleosomes, fibers and proteins. "Oleosomes", also known as "oil bodies", "lipid bodies", "lipid droplets" or "spherosomes" are pre-emulsified droplets or vesicles that are by nature present in cells or plant seeds for storage of oil. They are used as energy source for plant growth and metabolism.

Typically, sunflower seeds are cleaned and/or dehulled prior to grinding.

The sunflower seeds are grinded using a mill such as, but not limited to, a ball-mill, a toothed colloid mill, a stone mill, or a rotor-stator mill. For grinding, an aqueous solution is added to the sunflower seeds in a ratio water to dry seeds of from 15/1 to 3/1, from 12/1 to 4/1 or from 10/1 to 5/1 prior to the grinding. Preferably, grinding is occurring at a temperature in a range of from 4 to 20°C, from 4 to 10°C, or from 4 to 8°C.

In one aspect of the invention, the grinding of the sunflower seeds in step a) is preceded by soaking and/or washing of the sunflower seeds.

The sunflower seeds may be allowed to soak in aqueous solution for a period of from 0.5 to 48 hours, from 0.75 to 24 hours, or from 1.0 to 12 hours. The sunflower seeds may be agitated during the period of soaking. The sunflower seeds are subsequently grinded together with the aqueous solution. Preferably, soaking is occurring at a temperature in a range of from 4 to 20°C, from 4 to 10°C, or from 4 to 8°C.

Optionally, following the period of soaking and prior to grinding, the aqueous solution may be removed, and the sunflower seeds may be washed one or more times by adding fresh aqueous solution. The sunflower seeds are subsequently grinded together with aqueous solution.

The aqueous solution, that is used in the preparations of the sunflower seed slurry, *i.e.* for grinding, soaking and/or washing, is water. Optionally, the aqueous solution may be an alkaline aqueous solution with a pH of from 7.0 to 7.2, or from 7.1 to 7.2. Alkaline aqueous solutions, such as, but not limited to, sodium hydroxide or sodium bicarbonate, may be used.

The sunflower seed slurry obtained from step a) of the process according to the invention has a pH of from 7.0 to 7.2, or from 7.1 to 7.2 prior to step b).

The sunflower seed slurry having a pH of from 7.0 to 7.2 may be obtained by adding a concentrated alkali solution to the sunflower seed slurry. Alternatively, the sunflower seed slurry having a pH of from 7.0 to 7.2 may be obtained by using an alkaline solution for grinding the sunflower seeds and, optionally, by further adjusting the pH by adding a concentrated alkali solution. The sunflower seed slurry may have pH in a range of from 7.0 to 7.2, or from 7.1 to 7.2.

The process step of grinding in step a) of the process according to the invention results in a sunflower seed slurry having particles with a size in a range of from 5 to 200 micron, from 10 to 190 micron, or from 15 to 180 micron.

The sunflower seeds slurry is characterized in that it has, based on total dry matter:
- a protein content of from 12 to 28 wt.%, or from 15 to 25 wt.%, or from 17 to 23 wt.%, and
- a lipid content of from 55 to 70 wt.%, or from 58 to 68 wt.%, or from 60 to 65 wt.%, of which at least 70%, at least 80%, or at least 90% is contained in oleosomes, and
- a fiber content of from 5 to 11 wt.%, or from 6 to 10 wt.%, or from 7 to 9 wt.%.

In the current invention, the term "lipid" or "lipids" is encompassing tri-, di-, monoglycerides, fatty acids and phospholipids
In the present invention, the protein content of a composition, such as a sunflower seeds slurry, sunflower oleosome fraction, or a sunflower oleosome concentrate, is expressed as the total Nitrogen content of the composition multiplied by 6.25; said total Nitrogen content being determined according to the Dumas combustion method.

### Roasting

In one aspect of the invention, the sunflower seeds may be subjected to a roasting process prior to step a) of the process according to the invention. Roasting may be applied by means of hot air at a temperature of from 110 to 145°C for a period of from 15 to 60 minutes, from 115 to 135°C for a period of from 20 to 45 minutes, or from 120°C to 130°C for a period of from 30 to 50 minutes. While roasting, a relative humidity in a range of from 10 to 30%, from 12 to 25%, or from 15 to 20% may be applied. This level of humidity may be achieved by injection of steam while roasting.

Roasting of the sunflower seeds may result in a pleasant taste and/or flavour of the oleosome concentrate obtained in the process according to the invention. The taste of such oleosome concentrate from sunflower seeds that has not been subjected to a roasting step may be described as a more vegetable, greenish taste. By subjecting the sunflower seeds to a roasting process, the vegetable, greenish taste may be reduced or even removed from the oleosome concentrate from these sunflower seeds. A more neutral taste is obtained that may, depending on the food application wherein the sunflower oleosome concentrate is used, be perceived as more pleasant. However, these sunflower oleosome concentrates have substantially no or minor typical flavors associated with roasting.

### Step b) Isolating a sunflower oleosome fraction

In step b) of the process of the invention a sunflower oleosome fraction is isolated from the sunflower seed slurry having a pH of from 7.0 to 7.2. Preferably, isolation of the sunflower oleosome fraction is occurring at a temperature in a range of from 4 to 20°C, from 4 to 10°C, or from 4 to 8°C.

For isolating the sunflower seed fraction, the sunflower seed slurry obtained from step a) may be separated into a solid fraction and a liquid fraction. Separation may be performed by means of filtration, centrifugation, or decantation. The solid fraction obtained is a fraction that is rich in fibers (sunflower cake). The liquid fraction is containing proteins and oleosomes.

The obtained liquid fraction is subsequently further separated by applying centrifugal acceleration which separates the filtrate into two liquid phases, a watery phase, *i.e.* a protein-rich fraction (sunflower milk) and an oily oleosome containing phase (sunflower cream) (i.e. the sunflower oleosome fraction).

Alternatively, for isolating the sunflower seed fraction, in step b) of the process the sunflower seed slurry from step a) may be submitted to a liquid-solid-liquid separation (three-phase separation) using a centrifugal tricanter. Such a separation is resulting in three fractions: the sunflower oleosome fraction (sunflower cream), a fiber-rich fraction (sunflower cake), and a protein-rich fraction (sunflower milk).

In one aspect, the process according to the present invention is performed in the absence of organic solvent. The organic solvent may be acetone, hexane, ethanol and the like.

### Step c) Washing the sunflower oleosome fraction and isolating the sunflower oleosome concentrate

In step c), of the process of the invention, the sunflower oleosome fraction is washed at a pH in a range of from 6.6 to 7.0, from 6.6 to 6.9, or from 6.7 to 6.8, and a sunflower oleosome concentrate is subsequently isolated.

For washing, the sunflower oleosome fraction is resuspended in an aqueous solution, such as water or a buffer solution like for example a sodium bicarbonate (NaHCO₃) buffer solution. The dry matter content of the resuspended sunflower oleosome fraction may be in a range of from 7 to 15 wt. %, from 8 to 14 wt.% of from 9 to 13 wt.%. The pH of the washed sunflower oleosome fraction may be adjusted using an aqueous acid solution such as a 10% solution of hydrochloric acid, sulphuric acid, lactic acid, citric acid or the like. Subsequently, the sunflower oleosome concentrate is isolated from the resuspended sunflower oleosome fraction by applying centrifugal acceleration. The centrifugal acceleration results into two liquid phases, a watery phase, *i.e.* a protein-rich fraction and an oily oleosome containing phase, i.e. the sunflower oleosome concentrate. The washing step may be conducted 1 up to 4 times.

In one aspect of the invention, the sunflower oleosome fraction is washed in an aqueous salt solution having mono- and/or bi-valent cations such as potassium ions (K⁺), sodium ions (Na⁺), magnesium ions (Mg²⁺) or calcium ions (Ca²⁺). Suitable salt solutions may be aqueous solutions of sodium chloride, potassium chloride, magnesium chloride, calcium chloride, sodium acetate, potassium acetate, sodium citrate, potassium citrate, or the like, and combination of two or more thereof. Preferably, the aqueous salt solution is selected from the group consisting of sodium chloride, potassium chloride, magnesium chloride, calcium chloride, sodium acetate, potassium acetate, sodium citrate, potassium citrate and combinations of two or more thereof. More preferably, the sunflower oleosome fraction is washed in an aqueous salt solution of sodium bicarbonate.

The aqueous salt solution for washing the sunflower oleosome fraction may have a concentration in a range of from 0.0.005 to 0.20 M, from 0.0.01 to 0.18 M, or from 0.02 to 0.15 M.

In one aspect of the invention, the process does not comprise an addition of reducing agents and/or acidulants. Examples of reducing agent and/or an acidulant are ascorbic acid, erythorbic acid, allyl isothiocyanate, cysteine, glutathione, dithiothreitol, sodium bisulfite, citric acid, malic acid, carnosic acid, any derivative thereof and any combination of two or more thereof.

The sunflower oleosome concentrate obtained from step c) of the process according to the invention is characterized in that it has based on total dry matter a protein content of from 8.0 to 17.0 wt.%, from 9.0 to 16.5 wt.%, from 13.0 to 16.0 wt.%.

The sunflower oleosome concentrate obtained from step c) of the process according to the invention is characterized in that it has, based on total dry matter:
- a protein content of from 8.0 to 17.0 wt.%, from 9.0 to 16.5 wt.%, from 13.0 to 16.0 wt.%, and
- a lipid content of from 83.0 to 92.0 wt.%, from 83.5 to 91.0 wt.%, or from 84.0 to 87.0 wt.%, of which at least 70%, at least 80%, or at least 90% is present as oleosomes.

In other words, at most 30%, at most 20%, or at most 10% of the lipid content is present as free oil.

The sunflower oleosome concentrate may further comprise small amounts of remaining fibers in an amount of up to 0.5 wt.%, up to 0.4 wt.%, or up to 0.3 wt.%, and/or minerals, expressed as ash content in an amount of up to 0.7 wt.% on total dry matter.

The sunflower oleosome concentrate obtained from step c) of the process according to the invention has a protein to lipid ratio in a range of from 0.09 to 0.20, from 0.10 to 0.19, or from 0.12 to 0.18.

The oleosomes in the sunflower oleosome concentrate obtained from step c) of the process according to the invention have an average globule diameter (D50) in a range of from 1.0 to 2.0 micron (µm), from 1.1 to 1.8 micron, or from 1.2 to 1.6 micron.

In the current invention, the average globule diameter of the oleosomes is expressed as the D50-value (D50). The D50-value of oleosomes is the diameter below which 50% of the volume of oleosome particles lies, and it is expressed in micron (= micrometer, symbol: µm).

To measure the average globule diameter (D50-value) of the oleosomes, the oleosomes are considered spherical and in case of non-spherical oleosomes, the diameter is considered as being the largest dimension that can be measured between two opposite points on the surface thereof.

To be able to measure the globule diameter of the oleosomes with a Mastersizer 3000 from Malvern, the oleosomes need to be diluted such that an obscuration in the range of from 3.0 to 8.0%, preferably 4.0 to 6.0% is obtained. Obscuration within the Mastersizer is the amount of light blocked or scattered, by the particles. Therefore, the oleosomes are diluted in a buffer solution containing 10 mM sodium phosphate, pH 7.4, and 1.0 weight% sodium dodecyl sulphate (SDS). To give some guidance, about 0.2 wt% of oleosomes is diluted in the buffer solution and the dilution is further adjusted to obtain the aforementioned obscuration. Once this optimal obscuration is obtained, the globule diameter is measured, and the average globule diameter (D50) can be calculated. The actual method applied in the current application is provided in detail in the section "examples: measurement of average globule size".

The process according to the present invention results in a yield of the sunflower oleosome concentrate, in a range of from 27 to 55 dry weight%, 30 to 50 dry weight % or 35 to 45 dry weight %. The yield of oleosome concentrate is expressed as the dry weight of oleosome concentrate obtained from the 100 g dry weight of sunflower seed slurry.

The process according to the present invention results a sunflower oleosome concentrate having a white or creamy-white color. The sunflower oleosome concentrate according to the invention has color values according to CIELAB of:
- °h in a range of from 85 to 95, from 86 to 94, or from 87 to 93 and C* of from 8.0 to 14.0, from 8.2 to 13.8, or from 8.4 to 13.6; or
- °h above 95, above 96, or above 97, and C* below 10.0, below 9.8, or below 9.6; measured on sunflower oleosome concentrate standardized to a dry substance in a range of 30 to 35 % and a pH in a range of 6.9 to 7.1, using an illuminant D65, an observation angle of 10°, specular component excluded and a view diameter of 30mm.

Each color can be represented by numerous color spaces. These spaces are standardized by the Commision International d'Eclairage (CIE) under a series of standard conditions. CIELAB L*a*b* is a color space that is commonly used for the analysis of colorimeter information, where L* defines lightness or darkness, a* redness or greenness, and b* yellowness or blueness.

Hue (°h) and Chromaticity (C*) can be calculated based on measured L*a*b* values.

Hue angle (°h) refers to the degree of the dominant spectral component, such as red, green, and blue, and ranges from 0°to 360°. An angle of 0°or 360°represents red Hue. Angles of 90°, 180°, and 270° respectively represent yellow, green, and blue Hue.

Color measurements are performed on sunflower oleosome concentrate standardized to a dry substance in a range of 30 to 35 % and a pH in a range of 6.9 to 7.1, using an illuminant D65, an observation angle of 10° and an aperture size of 30mm.

It has now been found that by applying process according to the present invention, it is possible to obtain a sunflower oleosome concentrate having a protein content in a range of from 8 to 17 wt.% and having a creamy white color. It has been found that the process according to the present invention results in stable oleosomes and is causing no or only minor amounts of free oil being present in the sunflower oleosome concentrate. The process according to the present invention is also causing no or only minor size increase of the oleosomes compared to the size of naturally occurring sunflower oleosomes. The sunflower oleosome concentrate has a creamy, pleasant taste with less nutty flavor and less off tastes, such as but not limited to bitter, metallic or green off taste.

In comparison, the pH in existing processes for isolating oleosome concentrates is high, usually above pH 8.0, and a subsequent washing step at high pH causes significant losses of proteins. The protein content of the oleosome sunflower concentrate is essential for the stability of the oleosomes. Unstable oleosomes will easily release oil. The presence of free oil is for certain food applications detrimental towards desired functionalities, such as for example foam stability. Additionally, a sunflower oleosome concentrate that is obtained according to existing processes has a green color that is not desirable.

### Further treatment of the oleosome concentrate

In another aspect of the invention, the sunflower oleosome concentrate, may be subjected to a heat treatment. The heat treatment may be a pasteurization treatment or an ultrahigh-temperature (UHT) treatment. Pasteurization treatment involves heating the oleosomes at a temperature of 65°C to 70°C for 30 minutes in batch, or 80°C to 86°C for 15 to 30 seconds in a continuous-flow process (High temperature short time Pasteurization (HTST pasteurization)). UHT treatment involves heating of oleosomes at a temperature of 135°C to 150°C in a continuous-flow process and holding at that temperature for one or more seconds, up to 5 seconds, before cooling rapidly to room temperature.

Optionally, the sunflower oleosome concentrate or the heat-treated sunflower oleosome concentrate may be further subjected to a dehydration step by means of, but not limited to, spray drying or lyophilization.

### The sunflower oleosome concentrate of the present invention

The invention further relates to a sunflower oleosome concentrate that is characterized in that it has, based on total dry matter:
- a protein content of from 8.0 to 17.0 wt.%, from 9.0 to 16.5 wt.%, from 13.0 to 16.0 wt.%, and
- a lipid content of from 83.0 to 92.0 wt.%, from 83.5 to 91.0 wt.%, or from 84.0 to 87.0 wt.%, of which at least 70%, at least 80%, or at least 90% is present as oleosomes.

In other words, at most 30%, at most 20%, or at most 10% of the lipid content is present as free oil.

The sunflower oleosome concentrate may further comprise small amounts of remaining fibers in an amount of up to 0.5 wt.%, up to 0.4 wt.%, or up to 0.3 wt.%, and/or minerals, expressed as ash content in an amount of up to 0.7 wt.% on total dry matter.

The proteins of the isolated sunflower oleosome concentrate comprise proteins such as, but not limited to, "intrinsic proteins".

Said intrinsic proteins are mostly oleosin. Caleosin and stereolosin are minor intrinsic proteins. The oleosins contain a hydrophilic part, which is present at the isolated oleosomes' surface and a hydrophobic part which is anchored in the lipids and ensures for oleosome stability. Even at alkaline conditions of pH 8 or higher, proteins remain strongly bound, whereas weakly bound proteins will be removed in alkaline conditions.

The sunflower oleosome concentrate obtained according to the invention has a protein to lipid ratio in a range of from 0.09 to 0.20, from 0.10 to 0.19, or from 0.12 to 0.18.

The oleosomes in the sunflower oleosome concentrate have an average globule diameter (D50) in a range of from 1.0 to 2.0 micron (µm), from 1.1 to 1.8 micron, or from 1.2 to 1.6 micron.

The sunflower oleosome concentrate is characterized in that it has a creamy-white color. The sunflower oleosome concentrate has a color, expressed in color values according to CIELAB of:
- °h in a range of from 85 to 95, from 86 to 94, or from 87 to 93 and C* of from 8.0 to 14.0, from 8.2 to 13.8, or from 8.4 to 13.6; or
- °h above 95, above 96, or above 97, and C* below 10.0, below 9.8, or below 9.6; measured on sunflower oleosome concentrate standardized to a dry substance in a range of 30 to 35 % and a pH in a range of 6.9 to 7.1, using an illuminant D65, an observation angle of 10°, specular component excluded and a view diameter of 30mm.
In one aspect of the invention, the sunflower oleosome concentrate does not comprise a reducing agent and/or acidulant.

The sunflower oleosome concentrate according to the invention has a protein content and a protein to lipid ratio that is higher compared to sunflower oleosome concentrates that are obtained by using existing processes. Without wanting to be bound by any theory, this higher protein content and higher protein to lipid ratio may result in an increased robustness of the oleosomes in the sunflower oleosome concentrate. The sunflower oleosome concentrate according to the invention may be more resistant to shear forces that are occurring during certain processes in the preparation of products such as food, feed and/or cosmetic products. Free oil may be released less easily from the sunflower oleosome concentrate according to the invention which may be beneficial for producing food products. An increased foaming capacity and shear resistance of the sunflower oleosome concentrate according to the invention is observed, which may be beneficial for producing aerated products.

Additionally, due to its higher protein content, compared to sunflower oleosome concentrates that are obtained by using existing processes, the sunflower oleosome concentrate according to the invention has a more interesting nutritional profile and is a better source of proteins in nutritional food compositions. As a consequence, less additional proteins, such as protein isolates need to be added to a food composition to reach the desired protein content. Such additional protein isolates often result in a pronounced vegetable taste that is perceived as unpleasant.

Furthermore, the higher protein content of the sunflower oleosome concentrate according to the invention, results in better sensorial quality. The sunflower oleosome concentrate has amongst others a more creamy, less watery mouthfeel compared to sunflower oleosome concentrates obtained using existing processes and having a lower protein content.

### Process for preparing products comprising the sunflower oleosome concentrate

The invention also relates to a process for preparing food and feed products, pharmaceutical products, personal care products, nutritional compositions and industrial products, and the process is comprising the step of blending the sunflower oleosome concentrate according to the invention with at least one ingredient other than oleosomes.

Examples of such food and feed products include but are not limited to drinks such as coffee, black tea, powdered green tea, cocoa, juice etc.; milk component-containing drinks, such as raw milk, processed milk, lactic acid beverages, etc.; a variety of drinks including nutrition-enriched drinks, such as calcium-fortified drinks and the like and dietary fiber-containing drinks, etc.; chilled or ambient dairy products, such as butter, cheese, vegan cheese, yoghurt, coffee whitener, whipping cream, custard cream, custard pudding, etc.; processed fat food products, such as mayonnaise, margarine, spread, shortening, etc.; soups; stews; seasonings such as sauce, dressings, etc.; a variety of paste condiments represented by kneaded mustard; a variety of fillings typified by jam and flour paste; a variety or gel or paste-like food products including red bean-jam, jelly, and foods for swallowing impaired people; food products containing cereals as the main component, such as bread, noodles, pasta, pizza pie, corn flake, etc.; Japanese, US and European cakes, candy, cookie, biscuit, hot cake, chocolate, rice cake, etc.; kneaded marine products represented by a boiled fish cake, a fish cake, etc.; live-stock products represented by ham, sausage, hamburger steak, etc.; daily dishes such as cream croquette, paste for Chinese foods, gratin, dumpling, etc.; foods of delicate flavor, such as salted fish guts, a vegetable pickled in sake lee, etc.; liquid diets such as tube feeding liquid food, etc.; supplements; and pet foods.

Whereas traditional processes for preparing food and feed products will require an emulsification or homogenization step, the process of the current invention for preparing the food and feed products does not need such a homogenisation or emulsification step. A simple blending of the sunflower oleosome concentrate with the other ingredients is sufficient.

Pharmaceutical products according to the invention may be formulated to include therapeutic agents, diagnostic agents and delivery agents. The pharmaceutical product will additionally contain an active ingredient as a therapeutic or diagnostic agent. The active ingredient can be anything that one wishes to deliver to a host. The active ingredient may be a protein or peptide that has therapeutic or diagnostic value. Such peptides include antigens (for vaccine formulations), antibodies, cytokines, blood clotting factors and growth hormones. An example of pharmaceutical product is a parenteral emulsion containing the sunflower oleosome concentrate, and a drug.

Personal care products according to the invention include soaps, cosmetics, skin creams, facial creams, toothpaste, lipstick, perfumes, make-up, foundation, blusher, mascara, eyeshadow, sunscreen lotions, hair conditioner, and hair coloring.

Industrial products according to the invention include paints, coatings, lubricants, films, gels, drilling fluids, paper sizing, latex, building and road construction material, inks, dyes, waxes, polishes and agrochemical formulations.

Nutritional compositions according to the invention may be compositions that are developed to cover the nutritional needs, either as a supplement, or as a complete nutrition. The people that are targeted for the nutritional composition according to the invention relate to specific groups of people, such as, but not limited to, preterm infants, infants, toddlers, elderly people, athletes or humans having nutritional deficiencies and/or having a deficient immune system. They may be designed for people suffering a more specific disease state such as cancer, chronic obstructive pulmonary disease, and later-stage kidney disease and others. Amongst others, nutritional compositions may be helpful for people who struggle with a loss of appetite, have difficulty chewing, have trouble preparing balanced meals, and/or are recovering from surgery or an illness. **In** the event that the nutritional composition is meant for a complete nutrition, it can provide a healthy balance of protein, carbohydrate, and/or fat.

These nutritional compositions can be in the form of liquid, as a ready-to-drink formula or used in feeding tubes. It can also be in the form of a formula base i.e. a powder or a concentrated liquid, to be dissolved in water or in another fluid for the preparation of a ready-to-drink nutritional composition. The nutritional composition may also be in the form of a pudding or a jelly, or in form of a cookie or a snack bar, or in any other form.

In one aspect of the invention the nutritional composition is comprising the sunflower oleosome concentrate in an amount of from 1 to 70 weight % on dry matter of the nutritional composition. The nutritional composition is comprising the sunflower oleosome concentrate in an amount of from 5 to 65 weight %, from 10 to 60 weight %, from 15 to 55 weight %, from 20 to 50 weight %, or from 25 to 45 weight % on dry matter of the nutritional composition.

The at least one ingredient other than oleosomes may be, but is not limited to proteins, carbohydrates, fats, vitamins, minerals, trace elements, essential amino acids, essential fatty acids, and mixtures of two or more thereof. Preferably, the at least one ingredient is selected from the group consisting of proteins, carbohydrates, fats, essential amino acids, essential fatty acids, vitamins, and mixtures of two or more thereof.

In another aspect of the invention the nutritional composition further comprises at least one non-nutritional ingredients.

Non-nutritional ingredients according to the invention are ingredients that do not substantially add to the caloric intake and/or do not substantially provide micronutrients. Examples of non-nutritional ingredients are flavors, colorants, emulsifiers, acid regulators such as citric acid or lactic acid, preservatives, and the like. The non-nutritional ingredients may be from a natural or synthetic origin.

In yet another aspect of the invention, the at least one ingredient other than oleosomes is not an emulsifier.

### EXAMPLES

### Step a): Obtaining a sunflower seed slurry having pH of from 7.0 to 7.2

200 g of dehulled seeds from high-oleic sunflower were soaked overnight in de-ionized water (ratio 1:2 seeds:water) at 4°C. The soaking water was discarded, and the soaked seeds were washed with de-ionized water at 4°C (ratio 1:2 seed: water). The dry substance (DS%) of the washed seeds was adjusted to 18% with de-ionized water at 4°C and subsequently milled in a Vorwerk Thermomix for 90 seconds at 10700 rpm. A sunflower seed slurry was obtained.

The pH of the sample of sunflower seed slurry was adjusted with NaOH (1M) to a pH of 7.2. The sample was left under magnetic stirring for 1 hour.

### Step b): Isolating a sunflower oleosome fraction

The sunflower seed slurry was filtered over a nylon filter with a pore diameter of 80 µm. The filtrate was collected and subsequently centrifuged for 1h at 4700 rpm and 4°C using a Thermo Scientific Legend XFR Centrifuge. The centrifugation process separated the liquid phase further into two liquid phases: a hydrophilic phase (supernatant) which was a watery solution of proteins, carbohydrates and soluble fibers, and a hydrophobic phase (creamy top layer) which was the desired sunflower oleosome fraction. The desired sunflower oleosome fraction was collected.

### Step c): Washing the sunflower oleosome fraction and isolating the sunflower oleosome concentrate

The sunflower oleosome fraction was subsequently washed in a buffer solution of 0.03M sodium bicarbonate. The pH was further adjusted using HCl (1M) to a pH of 6.8. The sample was left under magnetic stirring for 1 hour.
The samples were subsequently centrifuged for 1h at 4700 rpm and 4°C using a Thermo Scientific Legend XFR Centrifuge. After centrifugation, the creamy top layer was collected, i.e. the sunflower oleosome concentrate.

### Colour measurement

For colour measurement, the oleosome concentrate is standardized to a pH in a range of 6.9 to 7.1 and a dry matter content of 35% prior to color measurement.
L*a*b* was measured in a Konica Minolta spectrophotometer CM-5 with the following measurement settings:
- Measurement type: reflectance
- View diameter: 30 mm
- Specular component excluded (SCE)
- Auto-measurement: 3 times
- Observer angle: 10°
- Illuminant: D65

### Measurement of average globule size

The sunflower oleosome concentrate was re-dispersed or diluted in a buffer solution containing 10 mM sodium phosphate, pH 7.4, and 1.0 % sodium dodecyl sulphate (SDS). The average globules size, expressed as the D50 value, was measured using a Malvern Mastersizer 3000 equipped with a Hydro module. The concentration of the oleosomes in the buffer is such that an obscuration in the range of 8 to 8.5% in the Mastersizer equipment was obtained. A refractive index of 1.47 was used to measure the oleosomes size.

### Protein measurement

The protein content of the oleosomes was determined by the amount of Nitrogen in the sample. This amount of Nitrogen was analyzed using a combustion method according to Dumas. Combustion of the sample was performed at 1100 °C. The amount of Nitrogen was determined using a conductivity detector (LECO TruMAc).
The protein content was calculated by multiplying the amount of Nitrogen analyzed by 6.25.

### Results

Table 2 shows the protein content, lipid content as well as the yield of the sunflower oleosome concentrate. The yield is expressed as the weight of the oleosome sunflower concentrate on the weight of the sunflower seed slurry.
Furthermore, table 2 shows the average globule diameter (D50), the colour measurement of the oleosome concentrate as well as the visual colour description.

**Table 2**

| | Sample |
|---|---|
| Protein content (expressed as wt.% on total dry weight) | 15.0% |
| Lipid content (expressed as wt.% on total dry weight) | 86.7% |
| Free lipids (visual inspection) | none |
| Protein/lipid ratio | 0.17 |

| Colour measurement | |
|---|---|
| L* | +81.50 |
| a* | +1.39 |
| b* | +9.93 |
| °h | 82.03° |
| C* | 10.03 |
| Visual colour description | Creamy white |
| D50 (micron) | 1.3 |

## Claims

1. A process for preparing a sunflower oleosome concentrate and the process is comprising the steps, in order, of:
a) Grinding sunflower seeds in the presence of an aqueous solution and obtaining a sunflower seed slurry having a pH of from 7.0 to 7.2, and
b) Isolating from the sunflower seed slurry a sunflower oleosome fraction, and
c) Washing the sunflower oleosome fraction at a pH in a range of from 6.6 to 7.0 and isolating the sunflower oleosome concentrate.

2. The process according to claim 1, wherein the sunflower seeds are sourced from high oleic and/or mid oleic sunflower seeds.

3. The process according to any one of the preceding claims, wherein the grinding of the sunflower seeds is preceded by soaking and/or washing of the sunflower seeds.

4. The process according to any one of the preceding claims, and the process is further comprising a step of subjecting the sunflower oleosome concentrate obtained in step c) to a heat treatment step and/or a dehydration step.

5. The process according to any one of the preceding claims, and the process does not comprise an addition of reducing agents and/or acidulants.

6. A sunflower oleosome concentrate comprising, expressed on dry weight of the oleosome concentrate:
- a protein content of from 8.0 to 17.0 wt.%, and
- a lipid content of from 83.0 to 92.0 wt.%, of which at least 70% is present as oleosomes; measured according to the method in the description,
wherein the sunflower oleosome concentrate has a color, expressed in color values according to CIELAB of:
• °h in a range of from 85 to 95, from 86 to 94, or from 87 to 93 and C* of from 8.0 to 14.0, from 8.2 to 13.8, or from 8.4 to 13.6; or
• °h above 95, above 96, or above 97, and C* below 10.0, below 9.8, or below 9.6;
measured on sunflower oleosome concentrate standardized to a dry substance in a range of 30 to 35 % and a pH in a range of 6.9 to 7.1, using an illuminant D65, an observation angle of 10° and an aperture size of 30mm.

7. The sunflower oleosome concentrate according to claim 6, wherein the sunflower oleosome concentrate has a protein to lipid ratio in a range of from 0.09 to 0.20.

8. The sunflower oleosome concentrate according to claim 6 or claim 7, wherein the sunflower oleosome concentrate has a fiber content up to 0.5 wt.%.

9. A process for preparing food and feed products, pharmaceutical products, personal care products, nutritional compositions and industrial products, and the process is comprising the step of blending the sunflower oleosome concentrate according to any one of claims 6 to 9 with at least one ingredient other than oleosomes.

## Patentansprüche

1. Verfahren zum Herstellen eines Sonnenblumenoleosomkonzentrats und wobei das Verfahren in dieser Reihenfolge die Schritte umfasst:
a) Mahlen von Sonnenblumenkernen in der Gegenwart einer wässrigen Lösung und Erhalten einer Sonnenblumenkernaufschlämmung, die einen pH-Wert von 7,0 bis 7,2 aufweist, und
b) Isolieren einer Sonnenblumenoleosomfraktion aus der Sonnenblumenkernaufschlämmung und
c) Waschen der Sonnenblumenoleosomenfraktion bei einem pH-Wert in einem Bereich von 6,6 bis 7,0 und Isolieren des Sonnenblumenoleosomenkonzentrats.

2. Verfahren nach Anspruch 1, wobei die Sonnenblumenkerne aus Sonnenblumenkernen mit hohem Ölsäuregehalt und/oder mittlerem Ölsäuregehalt gewonnen werden.

3. Verfahren nach einem der vorstehenden Ansprüche, wobei dem Mahlen der Sonnenblumenkerne ein Einweichen und/oder Waschen der Sonnenblumenkerne vorausgeht.

4. Verfahren nach einem der vorstehenden Ansprüche, und wobei das Verfahren ferner einen Schritt umfasst, bei dem das in Schritt c) erhaltene Sonnenblumenoleosomkonzentrat einem Wärmebehandlungsschritt und/oder einem Dehydratisierungsschritt unterzogen wird.

5. Verfahren nach einem der vorstehenden Ansprüche und wobei das Verfahren keine Zugabe von Reduktionsmitteln und/oder Säuerungsmitteln umfasst.

6. Sonnenblumenoleosomkonzentrat, umfassend, ausgedrückt als Trockengewicht des Oleosomkonzentrats:
- einen Proteingehalt von 8,0 bis 17,0 Gew.-% und
- einen Lipidgehalt von 83,0 bis 92,0 Gew.-%, wovon mindestens 70 % als Oleosome vorliegen; gemessen nach dem Verfahren in der Beschreibung,
wobei das Sonnenblumenoleosomkonzentrat eine Farbe aufweist, ausgedrückt in Farbwerten gemäß CIELAB von:
• °h in einem Bereich von 85 bis 95, von 86 bis 94 oder von 87 bis 93 und C* von 8,0 bis 14,0, von 8,2 bis 13,8 oder von 8,4 bis 13,6; oder
• °h über 95, über 96 oder über 97 und C* unter 10,0, unter 9,8 oder unter 9,6;
gemessen an Sonnenblumenoleosomkonzentrat, standardisiert auf eine Trockensubstanz in einem Bereich von 30 bis 35 % und einem pH-Wert in einem Bereich von 6,9 bis 7,1, unter Verwendung einer Lichtart D65, eines Beobachtungswinkels von 10° und einer Blendengröße von 30 mm.

7. Sonnenblumenoleosomkonzentrat nach Anspruch 6, wobei das Sonnenblumenoleosomkonzentrat ein Protein-Lipid-Verhältnis in einem Bereich von 0,09 bis 0,20 aufweist.

8. Sonnenblumenoleosomkonzentrat nach Anspruch 6 oder 7, wobei das Sonnenblumenoleosomkonzentrat einen Ballaststoffgehalt von bis zu 0,5 Gew.-% aufweist.

9. Verfahren zum Herstellen von Nahrungs- und Futterprodukten, pharmazeutischen Produkten, Körperpflegeprodukten, Nährstoffzusammensetzungen und Industrieprodukten und wobei das Verfahren den Schritt eines Mischens des Sonnenblumenoleosomkonzentrats nach einem der Ansprüche 6 bis 9 mit mindestens einem anderen Bestandteil als Oleosomen umfasst.

## Revendications

1. Procédé de préparation d'un concentré d'oléosomes de tournesol et le procédé comprenant les étapes, dans l'ordre, consistant à :
a) broyer des graines de tournesol en présence d'une solution aqueuse et obtenir une suspension de graines de tournesol ayant un pH compris entre 7,0 et 7,2, et
b) isoler une fraction d'oléosomes de tournesol à partir de la suspension de graines de tournesol, et
c) laver la fraction d'oléosomes de tournesol à un pH compris entre 6,6 et 7,0 et isoler le concentré d'oléosomes de tournesol.

2. Procédé selon la revendication 1, dans lequel les graines de tournesol proviennent de graines de tournesol à haute teneur en acide oléique et/ou à teneur moyenne en acide oléique.

3. Procédé selon l'une quelconque des revendications précédentes, dans lequel le broyage des graines de tournesol est précédé d'un trempage et/ou d'un lavage des graines de tournesol.

4. Procédé selon l'une quelconque des revendications précédentes, et le procédé comprenant en outre une étape consistant à soumettre le concentré d'oléosomes de tournesol obtenu à l'étape c) à une étape de traitement thermique et/ou à une étape de déshydratation.

5. Procédé selon l'une quelconque des revendications précédentes, et le procédé ne comprend pas un ajout d'agents réducteurs et/ou d'acidifiants.

6. Concentré d'oléosomes de tournesol comprenant, exprimé en poids sec du concentré d'oléosomes :
- une teneur en protéines de 8,0 à 17,0 % en poids, et
- une teneur en lipides de 83,0 à 92,0 % en poids, dont au moins 70 % sont présents sous forme d'oléosomes ; mesurées selon le procédé décrit dans la description,
dans lequel le concentré d'oléosomes de tournesol a une couleur, exprimée en valeurs de couleur selon CIELAB de :
• °h dans une plage de 85 à 95, de 86 à 94, ou de 87 à 93 et C* de 8,0 à 14,0, de 8,2 à 13,8, ou de 8,4 à 13,6 ; ou
• °h supérieur à 95, supérieur à 96 ou supérieur à 97 et C* inférieur à 10,0, inférieur à 9,8 ou inférieur à 9,6 ;
mesurée sur un concentré d'oléosomes de tournesol normalisé à une substance sèche comprise entre 30 et 35 % et à un pH compris entre 6,9 et 7,1, à l'aide d'un illuminant D65, d'un angle d'observation de 10° et d'une taille d'ouverture de 30 mm.

7. Concentré d'oléosomes de tournesol selon la revendication 6, dans lequel le concentré d'oléosomes de tournesol a un rapport protéines/lipides compris entre 0,09 et 0,20.

8. Concentré d'oléosomes de tournesol selon la revendication 6 ou la revendication 7, dans lequel le concentré d'oléosomes de tournesol a une teneur en fibres allant jusqu'à 0,5 % en poids.

9. Procédé de préparation de produits destinés à l'alimentation humaine et animale, de produits pharmaceutiques, de produits de soins personnels, de compositions nutritionnelles et de produits industriels, et le procédé comprenant l'étape consistant à mélanger le concentré d'oléosomes de tournesol selon l'une quelconque des revendications 6 à 9 avec au moins un ingrédient autre que les oléosomes.
